# EUROPÄISCHE PATENTSCHRIFT

(11) **EP 1 545 233 B1**
(45) Veröffentlichungstag und Bekanntmachung des Hinweises auf die Patenterteilung: **27.02.2008**
(21) Anmeldenummer: 03748023.3
(22) Anmeldetag: 13.09.2003
(51) Int. Cl.: A23G 4/06, A23G 3/36, A61K 9/00

(54) **CALCIUMSALZ ENTHALTENDER KAUGUMMI**
CHEWING GUM CONTAINING CALCIUM SALT
GOMME A MACHER CONTENANT UN SEL DE CALCIUM

(30) Priorität: 23.09.2002 DE 20214694 U
(43) Veröffentlichungstag der Anmeldung: 29.06.2005
(73) Patentinhaber: Sus Tech GmbH & Co. KG, 64287 Darmstadt (DE)
(72) Erfinder: SCHECHNER, Gallus, 64285 Darmstadt (DE); BRAUNBARTH, Carola, 64380 Rossdorf (DE); POTH, Tilo, 69469 Weinheim (DE); FRANKE, Holger, 64462 Ginsheim (DE)
(74) Vertreter: Ulbrich, Carola
(86) Internationale Anmeldenummer: PCT/EP2003/010212
(87) Internationale Veröffentlichungsnummer: WO 2004/028271

(56) Entgegenhaltungen:
- EP-A- 0 091 611
- EP-A- 0 414 932
- WO-A-02/49578
- WO-A-03/059082
- US-A- 4 327 077
- US-A- 5 693 334
- US-A- 5 882 631
- US-A1- 2001 033 831

## Beschreibung

Die Erfindung betrifft einen Kaugummi, dadurch gekennzeichnet, dass er Komposite aus schwer wasserlöslichem Calciumsalz und einer Proteinkomponente enthält, wobei das schwer wasserlösliche Calciumsalz eine Teilchengröße von 5 bis 300nm aufweist.

Speisereste, die nach dem Essen im Mund verbleiben, sind eine der Hauptursachen für das Auftreten von Karies. In den meisten Fällen wird auf das Putzen der Zähne nach dem Essen einfach verzichtet. Insbesondere der darin enthaltene Zucker dient als Nährstoff für Bakterien der Mundhöhle, die einerseits durch bakterielle Abbauprodukte (insbesondere organische Säuren wie Milch-, Ameisen- oder Essigsäure) und andererseits eine vermehrte Plaquebildung für die Entstehung von Karies verantwortlich sind.

Das Kauen von Kaugummi nach dem Essen soll der Bildung von kariesfördemden bakteriellen Abbauprodukten entgegenwirken. Dazu werden in diesen Kaugummis sogenannte Zuckeraustauschsstoffe, insbesondere Zuckeralkohole wie Sorbit, lsomalt und Xylit eingesetzt. Zwar wird durch den erhöhten Speichelfluss die Bildung von kariesverursachenden Säuren verhindert oder zumindest vermindert, die allgemeine Zahngesundheit können diese Kaugummis aber nur in begrenztem Maße verbessern.

Der Zahnschmelz sowie das Stützgewebe der Knochen bestehen überwiegend aus dem Mineral Hydroxylapatit. Die Zugabe von Calcium- und/oder Phosphatsalzen zu Kaugummis soll dazu dienen, die Remineralisierung des Zahnschmelzes zu verbessern.

Nachteilig an diesen Zusammensetzungen ist, dass durch die Zugabe von herkömmlichen gemahlenen, mikrokristallinen Calcium- und/oder Phosphatsalzen keine ausreichende Remineralisierung des Zahnmaterials zu erhalten ist.

In der US-A-5 882 631 werden Formulierungen für Zahnpasten oder Kaugummis beschrieben, die poröses Calciumcarbonat mit Teilchengrößen im Bereich von 0,05 bis 0,5 µm sowie gegebenenfalls Alkalimetallfluoride enthalten. Die EP-A-0 091 611 betrifft einen Antikaries-Kaugummi, der neben Natriumfluorid, Calciumcitrat, Tricalcium-bis-orthophosphat und einer Grundmasse ein Proteinprodukt (Zein G-200) enthält, um eine Ablagerung von Fluorid in der Knochensubstanz zu verhindern.

Die Aufgabe der vorliegenden Erfindung ist es, alternativ einen Kaugummi bereitzustellen, der neben einem guten Geschmack zusätzlich einen positiven Nutzen für die Zahngesundheit während und/oder kurz nach seinem Genuss aufweist.

Die Aufgabe wird gelöst durch einen Kaugummi, dadurch gekennzeichnet, dass er Komposite aus schwer wasserlöslichem Calciumsalz und einer Proteinkomponente enthält, wobei das schwer lösliche Calciumsalz eine Teilchengröße von 5 bis 300 nm aufweist und ausgewählt ist aus Fluorapatit, Hydroxylapatit, Fluor-dotiertem Hydroxylapatit und deren Gemischen.

Der erfindungsgemäße Kaugummi besteht aus Zuckerarten und/oder Zuckeralkoholen, Süßstoffen, Aromen, anderen geruch- und geschmack- oder konsistenzgebenden Zutaten, Farbstoffen sowie einer wasserunlöslichen, beim Kauen plastisch werdenden Kaumasse. Daneben können die Kaugummis auch Trennmittel (wie beispielsweise Talkum) enthalten.

Kaumassen sind Gemische aus konsistenzgebenden Stoffen, den natürlichen Gummen, das sind erstarrte Säfte (Exudate) aus tropischen Pflanzen wie Chicle, Gummi arabicum, Guttapercha, Karayagummi und Traganth, Kautschuk und den thermoplastischen Kunststoffen Butadien-Styrol-Copolymerisate, Isobutylen-Isopren-Copolymerisate, Polyethylen, Polyisobutylen, Polyvinylester der unverzweigten Fettsäuren von C₂ bis C₁₈ und Polyvinylether.

Als Plastifikatoren werden Harze und Balsame eingesetzt. Zu den natürlichen Stoffen zählen Benzoeharz, Dammarharz, Kolophonium, Mastix, Myrrhe, Olibanum, Perubalsam, Sandarrak, Schellack und Tolubalsam, zu den synthetischen Cumaron-Inden-Harz, Glycerin-Pentaerythritester der Harzsäuren des Kolophoniums und deren Hydrierungsprodukte.

Zur Beeinflussung der Elastizität finden Paraffine (natürliche und synthetische) sowie Wachse Verwendung. Bei den Wachsen gibt es solche aus dem pflanzlichen Bereich wie Carnabauwachs und solche tierischen Ursprungs wie Bienenwachs oder Wollwachs, daneben solche aus dem mineralischen Bereich wie mikrokristalline Wachse, wie auch chemisch modifizierte oder synthetische Wachse. Als Weichmacher dienen Emulgatoren (z.B. Lecithine oder Mono- und Diglyceride von Speisefettsäuren) und Ester wie Glycerinacetat sowie auch Glycerin.

Zur Regulierung der Kaumassenkonsistenz werden pflanzliche Hydrokolloide wie Agar-Agar, Alginsäure und Alginate, Guarkernmehl, Johannisbrotkernmehl oder Pektin zugesetzt. Zur gezielten Einstellung der Kaueigenschaften von Kaumassen werden Füllstoffe eingesetzt, das sind Carbonate von Calcium oder Magnesium, Oxide, beispielsweise Aluminiumoxid, Kieselsäure und Silicate von Calcium oder Magnesium. Stearinsäure und ihre Calcium- und Magnesiumsalze werden zur Verminderung des Haftvermögens der Kaumasse am Zahnschmelz eingesetzt.

Bevor die übrigen, für die Herstellung von Kaugummi rezepturgemäß erforderlichen Zutaten untergemischt werden, ist es erforderlich, die Kaumasse, die etwa 20-35 % (mindestens aber 15 %) des fertigen Kaugummis ausmacht, auf 50-60 °C zu erwärmen.

Durch die ausgeführte Kaubewegung fördert der Kaugummi den Speichelfluss. Die kariesverursachenden Säuren werden verdünnt und so auf natürliche Art die Gesundheit des Mundraums unterstützt.

Als in Wasser schwerlösliches Calciumsalz sollen solche Salze verstanden werden, die bei 20°C zu weniger als 0,1 Gew.% (1g/l) in Wasser löslich sind. Solche geeigneten Salze sind Calciumhydroxyphosphat (Ca₅[OH(PO₄)₃]) bzw. Hydroxylapatit, Calciumfluorphosphat (Ca₅[F(PO₄)₃]) bzw. Fluorapatit, und fluordotierter Hydroxylapatit der Zusammensetzung Ca₅(PO₄)₃(OH,F).

Als mineralisierender Wirkstoff eignet sich erfindungsgemäß ein feinteiliges, in Wasser schwerlösliches Calciumsalz, welches ausgewählt ist aus Fluorapatit, Hydroxylapatit, Fluor-dotiertern Hydroxylapatit und deren Gemischen. Diese Calciumsalze können sich am besten am im Zahnmaterial anlagern und eine Mineralisierung desselben bewirken.

Das wenig bzw. schwer wasserlösliche Calciumsalz weist erfindungsgemäß eine Teilchengröße bzw. Teilchenfeinheit von 5 bis 300 nm auf. Als Teilchenfeinheit soll hier der Durchmesser der Teilchen in Richtung ihrer größten Längenausdehnung verstanden werden. Die mittlere Teilchenfeinheit bezieht sich auf einen volumengemittelten Wert. Die erfindungsgemäßen Nanopartikel besitzen ein größeres Obertlächen/Volumenverhältnis als die mikrokristallinen Teilchen und zeichnen sich durch eine höhere Reaktivität im Vergleich zu diesen aus. Sie können daher besser zur Remineralisierung von entmineralisiertem Zahnmaterial eingesetzt werden. Unter Remineralisierung ist in diesem Zusammenhang die Wiedereinlagerung von Ionen in Knochenmaterial, also das Auffüllen von Fehlstellen innerhalb des bestehenden Zahnhartgewebes, wie Schmelz und Dentin, zu verstehen.

Überraschenderweise wurde darüber hinaus gefunden, dass sich durch Zugabe der Komposite zusätzlich auf dem Zahn neue Schichten eines biomimetischen Materials bilden können. Dieses Material ist dem natürlichen Zahnhartgeweben chemisch und strukturell sehr ähnlich. Es werden daher nicht nur Fehlstellen innerhalb der Kristallstruktur ausgeglichen, wie es bei der Remineralisierung des Zahnmaterials geschieht, sondern auch neues, am Zahn anhaftendes, in seiner Nanostruktur dentinähnliches Material erzeugt. Diese Neuentstehung von biomimetischem Material wird im weiteren als Neomineralisierung bezeichnet werden.

Unter Kompositmaterialien werden erfindungsgemäß Verbundstoffe verstanden, die ein schwer wasserlösliches Calciumsalz und eine Proteinkomponente umfassen und mikroskopisch heterogene, makroskopisch aber homogen erscheinende Aggregate darstellen.

Die in den Kompositmaterialien vorliegenden feinteiligen Calciumsalz-Primärteilchen können auch von einem oder mehreren Oberflächenmodifikationsmitteln umhüllt sein.

Dadurch kann beispielsweise die Herstellung von Kompositmaterialien in solchen Fällen erleichtert werden, bei welchen sich die nanopartikulären Calciumsalze schwer dispergieren lassen. Das Oberflächenmodifikationsmittel wird an die Oberfläche der Nanopartikel adsorbiert und verändert sie dergestalt, dass die Dispergierbarkeit des Calciumsalzes zunimmt und die Agglomeration der Nanopartikel verhindert wird.

Darüber hinaus kann durch eine Oberflächenmodifikation die Struktur der Kompositmaterialien sowie die Beladung von weiteren Komponenten mit dem nanopartikulären Calciumsalz beeinflusst werden. Auf diese Weise ist es bei der Anwendung der Kompositmaterialien in Mineralisationsprozessen möglich, Einfluss auf den Verlauf und die Geschwindigkeit der Re- und Neomineralisierung (erfindungsgemäß unter dem Begriff Mineralisierung zusammengefasst) zu nehmen.

Unter Oberflächenmodifikationsmitteln sind Stoffe zu verstehen, welche an der Oberfläche der feinteiligen Partikel physikalisch anhaften, mit diesen jedoch nicht chemisch reagieren. Die einzelnen an der Oberfläche adsorbierten oder gebundenen Moleküle der Oberflächenmodifikationsmittel sind im wesentlichen frei von intermolekularen Bindungen untereinander. Unter Oberflächenmodifikationsmitteln sind insbesondere Dispergiermittel zu verstehen. Dispergiermittel sind dem Fachmann auch unter den Begriffen Tenside und Schutzkolloide bekannt. Geeignete Tenside oder polymere Schutzkolloide können der deutschen Patentanmeldung DE 198 58 662 A1 entnommen werden.

Die Herstellung der erfindungsgemäßen Kompositmaterialien, in welchen die Primärpartikel der Calciumsalze oberflächenmodifiziert sind, kann nach analogen Fällungsverfahren wie vorstehend beschrieben erfolgen, wobei jedoch die Fällung der nanopartikulären Calciumsalze oder der Kompositmaterialien in Gegenwart eines oder mehrerer Oberflächenmodifikationsmittel erfolgt:

Gemäß einer bevorzugten Ausführungsform weist das wenig bzw. schwer wasserlösliche Calciumsalz eine Teilchengröße bzw. Teilchenfeinheit von 5 bis 100 nm auf. Ein Vorteil dieser besonders geringen Teilchengrößen bzw. -feinheiten besteht darin, dass diese Primärteilchen überraschenderweise eine besonders effektive Remineralisierung der Zähne zeigen und darüber hinaus die Fähigkeit aufweisen, neue Schichten von dem Zahnhartgewebe sehr ähnlichem Material zu bilden.

Nach einer besonders bevorzugten Ausführungsform weisen die erfindungsgemäßen Calciumsalze eine längliche, insbesondere stäbchen- oder nadelähnliche Form auf. Dies hat den besonderen Vorteil, dass sie der Form der biologischen Apatite (z.B. Knochen- bzw. Dentinapatite) sehr ähnlich sind und daher eine besonders gute Fähigkeit zur Re- und Neomineralisierung aufweisen. Solche Calciumsalze lassen sich z.B. nach dem aus DE 198 58 662 A1 bekannten Verfahren in Form stäbchenförmiger Primärteilchen herstellen.

Gemäß einer bevorzugten Ausführungsform sind 0,01 bis 2 Gew.-% Komposite enthalten. Es ist bevorzugt insbesondere 0,1 bis 1 Gew.% Komposite in dem erfindungsgemäßen Kaugummi einzusetzen.

Proteine können an die Oberfläche der Nanopartikel adsorbiert werden, wodurch ein Kompositmaterial aus Protein und schwer wasserlöslichem Calciumsalz entsteht. Insbesondere wird durch die adsorbierten Proteine auch eine Koagulation und Agglomeration der Calciumsalze verhindert und das Kristallwachstum verlangsamt. Bei der Mineralisation eines Zahns, und insbesondere bei der Neomineralisierung ist es von großem Vorteil, wenn kein unkontrolliertes Kristallwachstum stattfindet, welches nur ein lockeres Kristallgefüge ausbilden könnte. Durch das Proteingerüst kann das Kristallwachstum gebremst und kontrolliert ablaufen. So wird ein besonders dichtes und festes Kristallgefüge gebildet.

Überraschenderweise wurde gefunden, dass die Komposite aus schwer wasserlöslichem Calciumsalz mit Proteinen neben einer Remineralisation des Zahns auch in der Lage sind, das Ausmaß größerer Schäden im Zahndentin und/oder Zahnschmelz durch die Bildung vollständig neuer Kristalle zu verringern.

Bei der natürlichen Entstehung von Knochenmaterial wie z.B. des Zahnschmelzes und -dentins bewirkt eine Proteinmatrix die geordnete Anlagerung von Hydroxylapatit im Zahn oder Knochen, die hauptsächlich aus Kollagen sowie anderen Proteinen besteht. Mit den Kompositen aus schwerlöslichem Calciumsalz und Proteinen verläuft die Neomineralisierung ähnlich wie die Biomineralisierung und führt damit zu einem besonders positiven Effekt auf die Zahngesundheit beim Genuss des erfindungsgemäßen Kaugummis.

Die in dem Komposit bevorzugt enthaltene Proteinkomponente ist insbesondere ausgewählt aus Proteinen, Proteinabbauprodukten und Derivaten von Proteinen oder Proteinabbauprodukten.

Als Proteine kommen dabei alle Proteine unabhängig von ihrer Herkunft in Frage, also sowohl tierische wie pflanzliche Proteine. Geeignete tierische Proteine sind z.B. Kollagen, Fibroin, Elastin, Keratin und Albumin. Geeignete pflanzliche Proteine sind z.B. Weizen- und Weizenkeimproteine (Gluten), Reisprotein, Sojaprotein, Haferprotein, Erbsenprotein, Mandelprotein und Kartoffelprotein. Auch Einzellerproteine wie z.B. Hefeprotein oder Bakterienproteine sind geeignet.

Erfindungsgemäß bevorzugte Proteine sind tierische Produkte wie Kollagen und Keratin. Das Protein kann aber ebenfalls aus einer pflanzlichen oder marinen Quelle ausgewählt sein.

Als Proteinabbauprodukte werden solche Produkte verstanden, die durch hydrolytischen, oxidativen oder reduktiven Abbau von wasserunlöslichen Proteinen zu Oligo- und Polypeptidstrukturen mit niedrigerem Molekulargewicht und mit einer verbesserten Wasserlöslichkeit erhältlich sind.

Der hydrolytische Abbau wasserunlöslicher Proteine ist die wichtigste Abbaumethode; sie kann unter dem katalytischen Einfluss von Säuren, Alkalien oder von Enzymen erfolgen. Bevorzugt geeignet sind vor allem solche Proteinabbauprodukte, die nicht weiter abgebaut sind als zur Erlangung der Wasserlöslichkeit erforderlich.

Zu den wenig abgebauten Proteinhydrolysaten zählt beispielsweise die im Rahmen der vorliegenden Erfindung bevorzugte Gelatine, welche Molmassen im Bereich von 15000 bis 400000 D aufweisen kann. Gelatine ist ein Polypeptid, das vornehmlich durch Hydrolyse von Kollagen unter sauren oder alkalischen Bedingungen gewonnen wird. Besonders bevorzugt ist unter sauren oder stark sauren Bedingungen gewonnene Gelatine. Die Gelstärke der Gelatine ist proportional zu ihrem Molekulargewicht, d. h. eine stärker hydrolysierte Gelatine ergibt eine niedriger viskose Lösung. Die Gelstärke der Gelatine wird in Bloom-Zahlen angegeben. Bei der enzymatischen Spaltung der Gelatine wird die Polymergröße stark erniedrigt, was zu sehr niedrigen Bloom-Zahlen führt.

Unter Derivaten von Proteinen und Proteinabbauprodukten werden chemisch modifizierte Proteine oder Proteinhydrolysate verstanden, die z.B. durch Acylierung freier Aminogruppen, durch Anlagerung von Ethylen- oder Propylenoxid und Hydroxyl-, Amino- oder Carboxylgruppen oder durch Alkylierung von Hydroxylgruppen des Proteins oder Proteinabbauproduktes oder eines Hydroxyalkylderivats davon, z.B. mit Epoxypropyl-trimethylammoniumchlorid oder 3-Chlor-2-hydroxypropyl-trimethylammoniumchlorid, erhältlich sind.

In einer besonders bevorzugten Ausführung ist die Proteinkomponente ausgewählt aus Gelatine, deren Hydrolysaten und Gemischen davon. Es sollte bevorzugt eine Proteinkomponente in einer Menge von wenigstens 1 Gew.-%, bevorzugt von 1 bis 50, insbesondere von 20 bis 40 Gew.-%, enthalten sein.

In den erfindungsgemäßen Kompositen liegen die Primärpartikel der Calciumsalze an das Gerüst der Proteinkomponente assoziiert vor. Der Anteil der Proteinkomponenten in solchen Kompositmaterialien liegt zwischen 0,1 und 50 Gew.% bevorzugt jedoch zwischen 1,0 und 45 Gew.-%, insbesondere 20 bis 40 Gew.% bezogen auf das Gewicht des Kompositmaterials.

Geeignet sind Kompositmaterialien, bei denen die feinteiligen schwerlöslichen Calciumsalze mit Teilchenfeinheiten von 5 bis 300 nm zusammen mit feinteiligen Proteinen, Proteinhydrolysaten oder Derivaten davon eine räumliche Struktur derart bilden, dass die feinteiligen Calciumsalze der Proteinstruktur aufgelagert sind, diese quasi räumlich abbilden. Aus solchen bevorzugt geeigneten nanopartikulären Calciumsalzen und Proteinkomponenten bestehende Kompositmaterialien führen zu einer besonders guten Mineralisierung der Zähne beim Genuss des erfindungemäßen Kaugummis.

Besonders gut können sich schwer wasserlösliche Calciumsalze in Stäbchenform an die Proteinketten anlagern. Dies führt zu einem deutlich verbesserten Zusammenhalt des Kompositmaterials. Geeignet sind dabei Primärpartikel mit einer Teilchenfeinheit von 5 bis 300 nm, und bevorzugt von 5 bis 100 nm, da diese besonders kleinen Kristallite der Form biologischer Apatite sehr ähnlich und sich auch wegen der geringen Größe noch besser an die Proteinketten anlagern können. Diese Komposite führen dadurch zu einer besonders effektiven Mineralisierung von Zähnen.

Erfindungsgemäß geeignete Kompositmaterialien können durch Fällung aus wäßrigen Lösungen wasserlöslicher Calciumsalze mit wäßrigen Lösungen wasserlöslicher Phosphat und/oder Fluoridsalze in Gegenwart von Proteinkomponenten nach unterschiedlichen Verfahren hergestellt werden, wie sie bereits in der deutschen Patentanmeldung DE 199 30 335 beschrieben sind.

Für die Herstellung der erfindungsgemäßen Kaugummis wird der Wirkstoff, also das Kompositmaterial aus dem schwerlöslichen Calciumsalz und einer Proteinkomponente, einfach zu der Zuckerlösung, zugegeben und eingerührt.

Gemäß einer besonderen Ausführungsform enthält der erfindungsgemäße Kaugummi Zucker und/oder Zuckeralkohole. Die dabei bevorzugt zu verwendenden Zuckerarten Mono-, Di- und Oligosaccharide wie bspw. Dextrose, Fructose und Saccharose, Glucosesirup, flüssige Zucker und verwandte Erzeugnisse, getrockneter Glucosesirup und andere Stärkeverzuckerungserzeugnisse sowie darüber hinaus auch Zuckeraustauschstoffe, insbesondere Zuckeralkohole, sein.

Trotz der zum Teil zahnschädigenden Inhaltsstoffe (Zucker) führt der Konsum der erfindungsgemäßen Kaugummis neben dem Genusserlebnis zur Zahnpflege und Zahnschonung sowie darüber hinaus zur Mineralisierung des Zahnschmelzes und/oder des Dentins. Auf die, nicht immer nach dem Essen mögliche, aber zur Gesunderhaltung der Zähne bisher nötige Zahnpflege, üblicherweise mit Zahnbürste, Zahnpasta und/oder Mundwasser, kann so ohne Schaden für die Zähne verzichtet werden.

Erfindungsgemäß bevorzugte Zuckeralkohole sind Sorbit bzw. Sorbitsirup, Mannit, Xylit, Lactit, Isomalt, Maltit bzw. Maltitsirup. Diese Stoffe haben den Vorteil, dass sie pro 100 g weniger Kalorien enthalten und darüber hinaus der Abbau der Zuckeralkohole zu Säuren durch einige Bakterien der Mundhöhle so langsam erfolgt, dass sie nicht kariogen wirken. Der erfindungsgemäße Zusatz von Kompositen in zuckeraustauschstoffhaltigen Kaugummis bewirkt eine Mineralisierung der Zähne während und/oder nach dem Genuss des Kaugummis und trägt somit besonders zum Erhalt gesunder Zähne bei.

Gemäß einer weiteren bevorzugten Ausführungsform enthält der Kaugummi neben den Kompositen zusätzlich mindestens ein Fluoridsalz. Überraschenderweise wurde gefunden, dass die Zugabe von Fluorid zu einer synergistischen Verstärkung des nukleierenden Effekts der Komposite führt. Insbesondere bevorzugt ist der Zusatz von Natrium- und/oder Kaliumfluorid. Bei gleichzeitiger Zugabe von Kompositen und geringen Mengen Fluorid zeigt sich eine etwa fünffache synergistische Verstärkung. Bevorzugt sind in den erfindungsgemäßen Kaugummis von 0,05 bis 0,15 Gew.%, insbesondere von 0,08 bis 0,12 Gew.-% Fluoridsalz enthalten.

Gemäß einer weiteren bevorzugten Ausführungsform enthält der Kaugummi Aromastoffe, Süßstoffe, Füllstoffe und/oder weitere Hilfsstoffe (wie z.B. Glyzerin oder Mineralsalze, bspw. Zn²⁺ oder Mg²⁺).

Grundsätzlich können jegliche natürlichen oder naturidentischen Aromastoffe eingesetzt werden. Besonders bevorzugt können insbesondere Aromaöle wie z.B. Pfefferminzöl, Krauseminzöl, Eukalyptusöl, Anisöl, Fenchelöl, Kümmelöl und synthetische Aromaöle eingesetzt werden.

Auch Fruchtaromen können, insbesondere in festen oder flüssigen Fruchtzubereitungen, Fruchtextrakten oder Fruchtpulvern, enthalten sein. Bevorzugt sind dabei Ananas, Apfel, Aprikose, Banane, Brombeere, Erdbeere, Grapefruit, Heidelbeere, Himbeere, Maracuja, Orange, Sauerkirsch, rote und schwarze Johannisbeere, Waldmeister und Zitrone.

Es können auch Wirkstoffe, wie z.B. Menthol und/oder Vitamine, in dem erfindungsgemäßen Kaugummi enthalten sein. Ebenso können Organophosphonate, wie z.B. 1-Hydroxyethan-1,1-diphosphonsäure, Phosphonopropan-1,2,3-tricarbonasäure (Na-Salz) oder 1-Azacycloheptan-2,2-diphosphonsäure (Na-Salz), und/oder Pyrophosphate zugesetzt werden, die die Bildung von Zahnstein vermindern.

Süßungsmittel wie z.B. Saccharin-Natrium, Acesulfam-K, Aspartame^{®}, NatriumCyclamat, Stevioside, Thaumatin, Sucrose, Lactose, Maltose, Fructose oder Glycyrrhicin sind ebenfalls bevorzugt enthalten. Damit kann der Anteil an Zucker reduziert und trotzdem der vorwiegend süße Geschmack erhalten werden.

Als Konservierungsstoffe können alle für Lebensmittel zugelassenen Konservierungsstoffe eingesetzt werden, bspw. Sorbin- oder Benzoesäure und deren Derivate, wie z.B. Natriumbenzoat und Parahydroxybenzoat (Natriumsalz), Schwefeldioxid oder schwefelige Säure, Natrium- oder Kaliumnitrit. Farbstoffe und Pigmente zur Erreichung eines ansprechenden Aussehens können ebenfalls enthalten sein.

Nach einer bevorzugten Ausführungsform ist der erfindungsgemäße Kaugummi gefüllt. In dieser Füllung können vorteilhafterweise verschiedene Substanzen eingearbeitet sein. Es ist beispielsweise möglich, in diese Füllung ausgewählte Aromen hinzuzufügen, die insbesondere das Geschmackserlebnis steigern. Auch Vitamine und andere Zusatzstoffe können in dieser Füllung eingeschlossen werden.

Diese Füllung kann insbesondere eine Lösung, eine Suspension, ein Gel oder ein Sirup sein. Insbesondere können die Lösungen, Suspensionen oder Gele auf Wasserbasis hergestellt sein, um eine gute Verträglichkeit zu gewährleisten. Ein Zusatz von lebensmittelgeeignete. Dispergier- oder Netzmitteln kann dazu dienen, die Komposite in der Suspension zu halten. Als Gelbildner eignen sich insbesondere organische Verdickungsmittel sowie deren Derivate.

Neben synthetischen organischen Verdickem sind besonders natürliche organische Verdicker, insbesondere Agar-Agar, Carrageen, Tragant, Gummi arabicum, Alginate, Pektine, Polyosen, Guar-Mehl, Johannisbrotbaum-Kernmehl, Stärke, Dextrine, Gelatine und Casein geeignet. Davon abgewandelte Naturstoffe sind ebenfalls bevorzugt, insbesondere Carboxymethylcellulose und andere Celluloseether, Hydroxyethylcellulose und Hydroxypropylcellulose sowie Kernmehlether. Synthetische organische Verdicker wie z.B. Polyether oder anorganische Verdicker wie Polykieselsäuren und/oder Tonmineralien (z.B. Montmorillonite, Zeolithe oder Kieselsäuren) können ebenfalls erfindungsgemäß eingesetzt werden.

Gemäß einer besonders bevorzugten Ausführungsform enthält die Füllung des erfindungsgemäßen gefüllten Kaugummis die Komposite. Der Wirkstoff wird dabei vorteilhafterweise in die feste, gelartige oder flüssige Füllung eingearbeitet, die durch Aufbeißen auf den Kaugummi im Mund aus dem Kaugummi austritt und den Wirkstoff freisetzt. Im Falle einer flüssigen oder gelförmigen Füllung vermischt sich diese sofort und besonders gut mit dem Speichel.

Die Freisetzung des sich in der Füllung befindlichen Wirkstoffs wird damit beschleunigt und ein etwaiges Verkleben des Wirkstoffs mit der Kaumasse verhindert. So wird der Wirkstoff in vergleichsweise hohen Mengen im Mund verfügbar gemacht, dass er vorteilhafterweise eine besonders effektive Re- und Neomineralisierung der Zähne gewährleistet.

Insbesondere wird der die Komposite enthaltende Kaugummi zur Zahnpflege und Zahnschonung sowie darüber hinaus zur Mineralisierung des Zahnschmelzes und/oder des Dentins eingesetzt. Einer kariösen Erkrankung der Zähne kann so durch die Verwendung des erfindungsgemäßen Kaugummis entgegengewirkt werden.

Neben der Genussbefriedigung kann so der erfindungsgemäße Kaugummi zusätzlich zur Kariesprophylaxe verwendet werden.

Folgende Beispiele sollen die Erfindung erläutern, ohne sie darauf zu beschränken:

### Beispiel:

### 1. Herstellung eines Apatit-Protein-Komposits:

Zur Herstellung des Apatit-Gelatine-Komposits wird 2000, ml demineralisiertes Wasser in einem auf 25 °C thermostatisierten 4 1 Becherglas vorgelegt, in denen 44.10 g (0.30 Mol) CaCl₂-2H₂O (Fisher Chemicals p.a.) gelöst werden. Getrennt davon werden in 350 ml demineralisiertem Wasser 35 g Gelatine (Typ A, DGF-Stoess, Eberbach) bei etwa 50 °C gelöst. Beide Lösungen werden vereinigt und stark mit einem Propellerrührer gerührt. Der pH-Wert wird mit verdünnter wässriger Base auf 7.0 eingestellt.

Zu dieser Gelatine- und Calciumsalzlösung werden unter starkem Rühren innerhalb von 120 min 300 mL einer 0.6 M (NH₄)₂HPO4-Lösung, die vorher auf pH 7.0 eingestellt wurde, mit einer automatisierten Zugabeapparatur gleichmäßig zugepumpt. Dabei wird der pH-Wert durch die geregelte Zugabe von verdünnter wässriger Base konstant auf pH 7.0 gehalten. Nach Beendigung der Zugabe wird weiter über 24 h gerührt.

Anschließend wird die Dispersion in Zentrifugenbecher gefüllt und der Feststoffanteil durch Zentrifugieren von der Lösung getrennt. Durch fünffaches Aufschütteln des Rückstandes in demineralisiertem Wasser und anschließendes erneutes Zentrifugieren werden die Salze weitgehend ausgewaschen, so dass kein Chlorid mehr nachweisbar ist.

### 2. Herstellung der Kaumasse:

Die Grundmasse wird auf 50 °C vorerhitzt und der Doppel-Sigma-Schaufel-Kneter auf 40 °C thermostatisiert. Die halbe Menge des Sorbits, das feste Mannit, die Grundmasse sowie das Lecithin werden miteinander 5 Minuten geknetet. Dann wird die Hälfte des Glycerins zugegeben. Nach weiteren 5 Minuten werden ein Viertel des Sorbits und die Hälfte des Glycerins zugefügt. Nach einer weiteren Minute Kneten werden ein Viertel von der Sorbitmenge sowie die andere Hälfte des Glycerins zugeben. Diese Masse wird noch 5 Minuten geknetet bevor der Maltitsirup zugegeben wird. Abschließend kommen nach weiteren 5 Minuten das Optamint^{®} zur Masse und werden noch weitere 5 Minuten verknetet.
Die Masse wird mit Talkum bestreut und noch im warmen Zustand auf 1.5 mm Dicke ausgerollt. Aus diesen Platten werden Streifen geschnitten. Die geschnittenen Platten wiegen etwa 2.5 bis 4.0 g und werden durch Pressen oder aber durch Rotieren in einem eine runde Form gebracht. Die so hergestellten Kaugummikerne werden dann der Dragierung unterzogen.

**Tabelle 2: Kaumasse**

| Inhaltsstoffe | |
|---|---|
| Kaugummi-Grundmasse Balear^{®}-T (Cafosa, Spanien) | 33 g |
| D-Sorbit (98% rein, Aldrich) | 57.50 g |
| Mannit-Pulver (ABCR-Chemikalien) | 5.00 g |
| Maltit-Sirup (85 Gew.-%) in Wasser (HDS-Chemie, Österreich) | 3.00 g |
| Glycerin, wasserfrei (Merck) | 2.00 g |
| Optamint® (Haarmann & Reimer GmbH) | 1.80 g |
| Lecithin (ACROS Organics) | 0.50 g |

### 3. Herstellung von Apatit-Protein-Komposithaltigen Gelen:

Apatit-Gelatine-Komposit, Wasser, Glycerin und Maltitsirup werden unter starkem Rühren (400 rpm) auf 70 °C erwärmt. In der warmen Lösung wird Lecithin gelöst, danach wird Optamint^{®} zugegeben. Carboxymethylcellulose, Xylitol und Sorbit werden in einem Becherglas trocken vorgemischt und dann portionsweise bei gleichzeitiger Erhöhung der Rührgeschwindigkeit auf 750 rpm zugegeben. Nach 20 Minuten Rühren gibt man 0,35 g Benzoesäure zu und hält die Temperatur weitere 10 Minuten auf 70 °C. Abschließend wird die gefällte Kieselsäure (Sipernat^{®} 320 DS, Degussa AG) zudosiert, gleichzeitig die Rührgeschwindigkeit bis auf 1100 rpm erhöht und die Temperatur weitere 10 Minuten gehalten.

### 4. Herstellung von fluoridhaltigen Gelen mit Apatit-Gelatine-Komposit:

Analog zur Herstellung der fluoridfreien Gele werden auch die fluoridhaltigen Gele hergestellt. Die Hälfte der Sorbitmenge wird wie oben beschrieben zugegeben, während die andere Hälfte in einem zweiten Becherglas mit dem Natriumfluorid vorgemischt und dann ebenfalls portionsweise unter Erhöhung der Rührgeschwindigkeit auf 730 rpm zugegeben wird.

**Tabelle 2: Gel**

| | Gel ohne Fluorid | Gel mit Fluorid |
|---|---|---|
| Carboxymethylcellulose, Na-Salz Blanose^{®} CMC 9M31XF (Hercules) | 1,25g | 1,25g |
| Apatit-Gelatine-Komposit (5,90 %) in Glycerin dispergiert | 2,55g | 2,55g |
| Glycerin, wasserfrei (Merck) | 69g | 69g |
| Maltitsirup (HDS-Chemie, Österreich) | 6,3g | 6,3g |
| Deionisiertes Wasser | 51,0g | 51,0g |
| Natriumfluorid (Merck) | - | 0,45g |
| D-Sorbit, 97 %; (Aldrich) | 94g | 94g |
| Xylitol (Sigma) | 11,1g | 11,1g |
| Kieselsäure, gefällt Sipernat^{®} 320 DS (Degussa AG) | 11,75g | 11,75g |
| Lecithin (ACROS organics) | 0,25g | 0,25g |
| Optamint^{®} (Haarmann & Reimer) | 0,25g | 0,25g |
| Konservierungsmittel: Benzoesäure (ACROS organics) | 0,35g | 0,35g |

### 5. pH-Messungen in künstlichem Speichel:

### 5.1 Methode:

Das Gel, das die Komposite enthält, wird in eine Salzlösung gegeben, deren Gehalt an anorganischen Salzen demjenigen von Körperflüssigkeiten wie Speichel, Blut oder Plasma entspricht (Simulated Body Fluid, SBF) und die dementsprechend bezogen auf die Ausfällung von Calciumphosphat übersättigt ist. Solche Zusammensetzungen können als Modell für Körperflüssigkeiten eingesetzt werden, wie bereits in Liu et. al, Cells and Materials (1997), 7, S. 41-51) beschrieben wurde.

Für die vorliegende Untersuchung wurde ein SBF ("Simulated Body Fluid") verwendet, die aus einer wässrigen Lösung folgender Salze besteht:

| | | | |
|---|---|---|---|
| Na⁺ | 14 mM | PO₄³⁻ | 4,7mM |
| K⁺ | 21 mM | Cl⁻ | 30 mM |
| Ca²⁺ | 1,8mM | | |

Bei 37°C wurden je 70 mg Gel in 25 ml des SBF gelöst und die anschließende pH-Wert-Veränderung mittels einer pH-Elektrode (Inlab 410, Mettler Toledo; Meßgerät: Consort, Multi Parameter Analyzer C833) verfolgt.

### 5.2 Messergebnisse:

Ein mit geringen Mengen an Fluoridsalz versetztes Apatit-Protein-Komposit-Gel zeigt einen deutlich höheren Abfall des pH-Wertes als ein Gel ohne das Fluoridsalz. Solche Gele können beispielsweise in gefüllten Bonbons oder Kaugummis eingesetzt werden.

Diese pH-Wertveränderungen sind mit einer durch die Komposite induzierten Calciumphoshat-Fällung aus dem künstlichen Speichel zu erklären, die entsprechend der folgenden Gleichung, anhand des abfallenden pH-Wertes in der "SBF" gemessen wird:

10 CaCl₂ + 6 Na₂HPO₄ + 2 H₂O → Ca₁₀(OH)₂(PO₄)₆ + 12 NaCl + 8 HCl

Die Wirkungsweise der schwerlöslichen Calciumsalze liegt (ohne auf diese Theorie beschränkt sein zu wollen) aufgrund der Ergebnisse nicht nur in der Bereitstellung von Calcium- und/oder Phosphationen zum Einbau in die Dentinkanälchen und den Zahnschmelz. Darüber hinaus ist das schwerlösliche Calciumsalz insbesondere in der Lage, aus den im natürlichen Speichel des Menschen in übersättigten Konzentrationen vorliegenden Calcium- und Phosphationen abzuscheiden. Diese Nukleationswirkung führt so zur einem Aufbau von neuem Zahnmaterial (Neomineralisierung), insbesondere des Zahnschmelzes und/oder des Dentins, aus den körpereigenen Reservoirs.

Vorteilhafterweise können daher die zuzugebenden Mengen an schwer wasserlöslichem Calciumsalz klein gehalten werden, ohne dass die Re- und Neomineralisierungseffekte sich abschwächt. Das partikuläre Calciumsalz kann also als Kristallisationskeim für die im natürlichen Speichel vorkommenden Calcium- und Phosphationen wirken.

## Patentansprüche

1. Kaugummi, **dadurch gekennzeichnet, dass** er Komposite aus schwer wasserlöslichem Calciumsalz und einer Proteinkomponente enthält, wobei das schwer lösliche Calciumsalz eine Teilchengröße von 5 bis 300 nm aufweist und ausgewählt ist aus Fluorapatit, Hydroxylapatit, Fluor-dotiertem Hydroxylapatit und deren Gemischen.

2. Kaugummi nach Anspruch 1, **dadurch gekennzeichnet, dass** das schwer lösliche Calciumsalz eine Teilchengröße von 5 bis 100 nm aufweist.

3. Kaugummi nach einem der vorstehenden Ansprüche, **dadurch gekennzeichnet, dass** das schwer wasserlösliche Calciumsalz in Form von stäbchenförmigen Kristallen vorliegt.

4. Kaugummi nach einem der vorstehenden Ansprüche, **dadurch gekennzeichnet, dass** er von 0,01 bis 2 Gew.%, insbesondere von 0,1 bis 1 Gew.-%, Komposite enthält.

5. Kaugummi nach einem der vorstehenden Ansprüche, **dadurch gekennzeichnet, dass** die Proteinkomponente ausgewählt ist aus Gelatine oder deren Hydrolysaten.

6. Kaugummi nach einem der vorstehenden Ansprüche, **dadurch gekennzeichnet, dass** er Zucker und/oder Zuckeraustauschstoffe enthält.

7. Kaugummi nach einem der vorstehenden Ansprüche, **dadurch gekennzeichnet, dass** er zusätzlich Fluoridsalze enthält.

8. Kaugummi nach einem der vorstehenden Ansprüche, **dadurch gekennzeichnet, dass** er Aromastoffe, Süßstoffe, Füllstoffe und/oder weitere Hilfsstoffe enthält.

9. Kaugummi nach einem der vorstehenden Ansprüche, **dadurch gekennzeichnet, dass** der Kaugummi gefüllt ist.

10. Kaugummi nach Anspruch 9, **dadurch gekennzeichnet, dass** die Füllung die Komposite enthält.

## Claims

1. Chewing gum, **characterized in that** it contains composites of slightly water-soluble calcium salt and a protein component, wherein the slightly soluble calcium salt has a particle size of 5 to 300 nm and is selected from fluorapatite, hydroxylapatite, fluorine-doped hydroxylapatite and mixtures thereof.

2. Chewing gum according to Claim 1, **characterized in that** the slightly soluble calcium salt has a particle size of 5 to 100 nm.

3. Chewing gum according to one of the preceding claims, **characterized in that** the slightly water-soluble calcium salt is present in the form of rod-shaped crystals.

4. Chewing gum according to one of the preceding claims, **characterized in that** it contains from 0.01 to 2% by weight, in particular from 0.1 to 1% by weight, of composites.

5. Chewing gum according to one of the preceding claims, **characterized in that** the protein component is selected from gelatin or hydrolysates thereof.

6. Chewing gum according to one of the preceding claims, **characterized in that** it contains sugar and/or sugar replacers.

7. Chewing gum according to one of the preceding claims, **characterized in that** it additionally contains fluoride salts.

8. Chewing gum according to one of the preceding claims, **characterized in that** it contains flavourings, sweeteners, fillers and/or other aids.

9. Chewing gum according to one of the preceding claims, **characterized in that** the chewing gum is filled.

10. Chewing gum according to Claim 9, **characterized in that** the filling contains the composites.

## Revendications

1. Gomme à mâcher, **caractérisée en ce qu'**elle contient des composites de sels de calcium difficilement soluble dans l'eau et d'un composant protéinique, le sel de calcium difficilement soluble présentant une granulométrie de 5 à 300 nm et étant choisi parmi la fluorapatite, l'hydroxylapatite, l'hydroxylapatite dopée au fluor et leurs mélanges.

2. Gomme à mâcher selon la revendication 1, **caractérisée en ce que** le sel de calcium difficilement soluble présente une granulométrie de 5 à 100 nm.

3. Gomme à mâcher selon l'une quelconque des revendications précédentes, **caractérisée en ce que** le sel de calcium difficilement soluble dans l'eau se présente sous la forme de cristaux en forme de bâtonnets.

4. Gomme à mâcher selon l'une quelconque des revendications précédentes, **caractérisée en ce qu'**elle contient des composites à concurrence de 0,01 à 2 % en poids, en particulier de 0,1 à 1 % en poids.

5. Gomme à mâcher selon l'une quelconque des revendications précédentes, **caractérisée en ce que** le composant protéinique est choisi parmi la gélatine ou ses hydrolysats.

6. Gomme à mâcher selon l'une quelconque des revendications précédentes, **caractérisée en ce qu'**elle contient du sucre et/ou des produits de remplacement du sucre.

7. Gomme à mâcher selon l'une quelconque des revendications précédentes, **caractérisée en ce qu'**elle contient en outre des sels de fluorures.

8. Gomme à mâcher selon l'une quelconque des revendications précédentes, **caractérisée en ce que** qu'elle contient des arômes, des édulcorants, des substances de remplissage et/ou d'autres adjuvants.

9. Gomme à mâcher selon l'une quelconque des revendications précédentes, **caractérisée en ce que** la gomme à mâcher contient une substance de remplissage.

10. Gomme à mâcher selon la revendication 9, **caractérisée en ce que** la matière de remplissage contient les composites.
